# FASCICULE DE BREVET EUROPEEN

(11) **EP 2 758 229 B1**
(45) Date de publication et mention de la délivrance du brevet: **08.11.2017**
(21) Numéro de dépôt: 12767040.4
(22) Date de dépôt: 06.09.2012
(51) Int. Cl.: B29D 11/00, G02B 3/08, G02C 7/02

(54) **LENTILLE A PLUSIEURS SURFACES A ZONES**
LINSE MIT MEHREREN SEGMENTIERTEN FLÄCHEN
LENS HAVING A PLURALITY OF SURFACES WITH ZONES

(30) Priorité: 20.09.2011 FR 1158339
(43) Date de publication de la demande: 30.07.2014
(73) Titulaire: Essilor International (Compagnie Générale d'Optique), 94220 Charenton-le-Pont (FR)
(72) Inventeur: FERMIGIER, Bruno, F-94220 Charenton le Pont (FR); GACOIN, Eric, F-94220 Charenton le Pont (FR)
(74) Mandataire: Cabinet Plasseraud
(86) Numéro de dépôt international: PCT/FR2012/051999
(87) Numéro de publication internationale: WO 2013/041791

(56) Documents cités:
- WO-A1-2011/079856
- FR-A5- 2 057 540
- GB-A- 1 154 360
- JP-A- 7 241 919
- JP-A- 57 109 618
- JP-A- 63 106 737
- US-A1- 2002 063 862

## Description

La présente invention concerne une lentille à plusieurs surfaces à zones. Elle concerne en particulier une amélioration des lentilles de Fresnel qui sont déjà proposées ou utilisées.

De façon connue, une lentille de Fresnel, qui représente une lentille à une ou plusieurs surfaces à zones et sauts de zones, peut posséder une puissance optique équivalente à celle d'une lentille à surfaces lisses, tout en ayant une épaisseur inférieure. La réduction d'épaisseur et la réduction de poids qui en résulte sont avantageuses pour certaines applications, parmi lesquelles sont les applications ophtalmiques.

Une lentille de Fresnel comporte, sur au moins une de ces surfaces optiques, des sauts de hauteur sagittale entre des zones adjacentes à l'intérieur de cette surface optique. En effet, pour une lentille de Fresnel de type réfractif, la puissance optique est produite par la courbure de la surface optique à l'intérieur de chaque zone, d'une façon qui est quasi-indépendante d'un décalage de la surface optique dans cette zone parallèlement à l'axe optique de la lentille. Pour cette raison, dans des zones de la lentille où son épaisseur serait trop importante, il est possible de décaler la surface de la lentille parallèlement à son axe optique pour en réduire l'épaisseur, en créant ainsi un saut de hauteur sagittale dans la surface optique à la limite de la zone concernée.

Toutefois, pour qu'une telle lentille de Fresnel possède une qualité optique suffisante, il est nécessaire que sa surface optique dans chaque zone de Fresnel puisse être réalisée avec précision conformément à une surface-cible, cette dernière pouvant correspondre à la lentille à surface lisse.

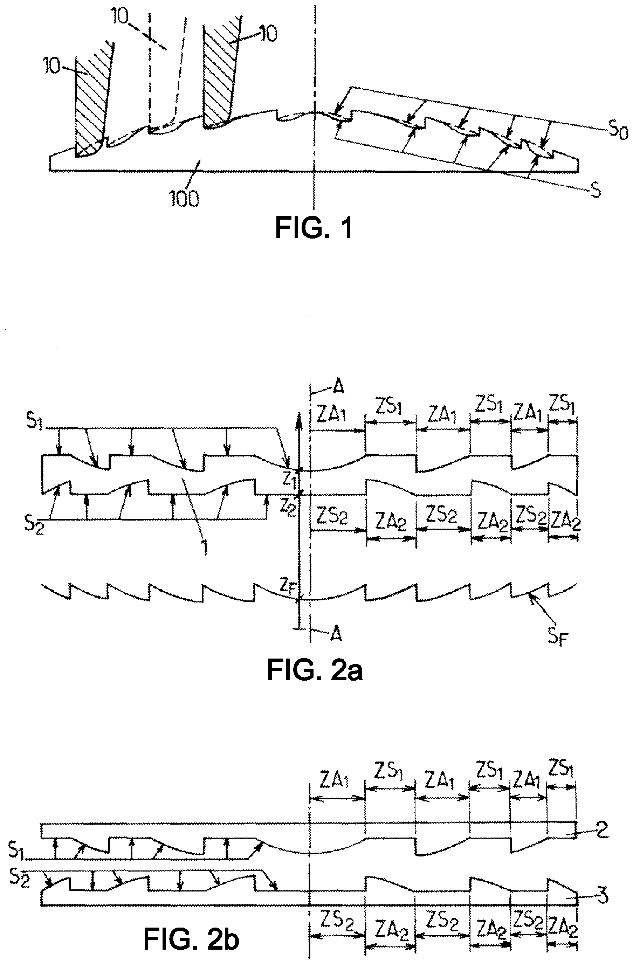

Or la surface à zones d'une lentille de Fresnel qui est constituée de matériau polymère peut être réalisée soit par embossage en utilisant des matrices d'embossage, soit par moulage en utilisant des inserts de moulage, soit par usinage direct d'une ébauche de la lentille de Fresnel. Pour les deux premiers procédés, les matrices d'embossage et les inserts de moulage comportent des surfaces-masters qui sont complémentaires de la surface à zones qui est voulue pour la lentille. La qualité optique de la lentille de Fresnel dépend donc de la possibilité réelle d'usiner chaque surface-master avec précision conformément à la surface-cible. Le plus souvent, les matrices d'embossage et les inserts de moulage sont métalliques, et usinés en déplaçant un outil d'enlèvement de matière contre leur surface. Mais cet outil possède à son extrémité de travail, un profil qui est déterminé pour lui procurer une rigidité et une robustesse suffisantes. Notamment, l'extrémité de travail de l'outil présente un angle de pointe et une longueur d'arête fixés, qui déterminent la taille minimale des motifs qui peuvent être usinés. Ces dimensions d'outil empêchent notamment d'obtenir exactement la surface-cible pour un surface-master de lentille de Fresnel au niveau d'une ligne d'angle rentrant entre deux zones adjacentes. La figure 1 illustre un tel empêchement, avec les références suivantes :
S₀ : surface-cible avec zones de Fresnel,
100 : matrice d'embossage ou insert de moulage,
10 : outil d'usinage à enlèvement de matière, pour trois positions de cet outil afin de réaliser les sauts de hauteur sagittale ; et
S : exemple de surface réellement obtenue par l'usinage.

La figure 1 représente la surface S qui est obtenue réellement si l'outil 10 est avancé jusqu'à parvenir au fond des angles rentrants qui sont situés à la base des sauts de hauteur sagittale, en enlevant un supplément de matière avec le dos de l'outil 10 qui est opposé à son angle d'attaque. A l'inverse, l'avancée de l'outil 10 peut être réduite pour ne pas dépasser la surface-cible S₀, mais dans ce cas les sauts de hauteurs sont raccourcis (voir la position de l'outil 10 qui est indiquée en traits interrompus).

Même si la matière de polymère qui constitue la lentille de Fresnel adopte ensuite exactement la forme du surface-master, lors de l'embossage ou du moulage, la qualité optique de la lentille résultante est limitée par l'écart d'usinage entre le surface-master et la surface-cible.

Cette limitation intervient de façon similaire pour une lentille de Fresnel dont la surface à zones est usinée directement.

En outre, les mêmes difficultés interviennent pour la réalisation d'une lentille diffractive qui possède aussi une surface à zones, avec des sauts de hauteur sagittale entre deux zones adjacentes.

Les documents JPS 57 109618 et GB 1 154 360 divulguent des lentilles de Fresnel qui sont symétriques entre leurs deux faces.

Le document FR 2 057 540 divulgue un système optique qui comporte au moins une surface de Fresnel et qui permet de former une image dépourvue de coma méridien.

Le document WO 2011/079856 divulgue un concentrateur de lumière pour cellule solaire, qui possède deux surfaces optiques à zones.

Le document JPS 63 106737 divulgue une lentille dont une face est pourvue d'une structure de Fresnel, et l'autre face est pourvue de bandes qui sont séparées par des sauts de hauteur sagittale.

Le document JPH 07 241919 concerne un outil de fabrication d'une surface de Fresnel.

Le document US 2002/063862 concerne un outil d'inspection et de contrôle pour des feuilles à lentilles de Fresnel.

Un but de la présente invention est alors de fournir des lentilles dont les surfaces optiques sont divisées en zones avec des sauts intermédiaires de hauteur sagittale, et qui présentent des qualités optiques améliorées. En particulier, l'invention a pour but de réduire les défauts de formage et d'usinage pour de telles lentilles à surfaces zonées.

Pour cela, l'invention propose une lentille qui comprend au moins deux surfaces optiques s'étendant chacune transversalement par rapport à un axe optique de la lentille, de sorte que des rayons lumineux qui traversent la lentille traversent l'une puis l'autre des deux surfaces, chaque surface comprenant des zones actives à l'intérieur desquelles une forme de cette surface est adaptée pour produire une partie au moins de la puissance optique de la lentille pour les rayons qui traversent ces zones actives, avec une discontinuité de hauteur sagittale de chaque surface entre deux zones actives différentes de cette surface. En outre, des limites des zones actives d'une première des deux surfaces sont situées au droit de limites des zones actives de l'autre des deux surfaces selon l'axe optique de la lentille.

Dans le cadre de l'invention, l'expression «au droit de» est utilisée sans distinction de sens avec «en vis-à-vis de», notamment dans le cas où l'invention est appliquée à une lentille qui présente une base cambrée. L'alignement qui est ainsi désigné par les expressions «au droit de» ou «en vis-à-vis de» fait référence à la direction de propagation des rayons lumineux entre les deux surfaces optiques de la lentille, pour une utilisation de celle-ci. En effet, la présente invention s'applique à un composant optique qui peut être soit :
- à base plane : pour chaque surface de la lentille, les sommets ou les fonds des sauts de hauteur sagittale sont situés au niveau d'un même plan qui perpendiculaire à l'axe optique. Dans ce premier cas, les limites des zones actives de la première des deux surfaces peuvent être situées au droit des limites des zones actives de l'autre des deux surfaces selon l'axe optique de la lentille ; ou
- à base cambrée : pour chaque surface de la lentille, les sommets ou les fonds des sauts de hauteur sagittale sont situés sur une sphère ou une surface asphérique qui est parallèle à une même surface de référence, appelée base. Dans ce second cas, les zones des deux surfaces peuvent être dites en vis-à-vis lorsqu'elles sont alignées selon une direction qui est perpendiculaire à la base.

En fait, ces deux cas sont équivalents par rapport à la forme de la base de la lentille.

Par ailleurs, dans chaque cas de base plane ou cambrée, les sauts de hauteur sagittale présentent chacun une direction de saut qui peut être parallèle à l'axe optique de la lentille ou inclinée par rapport à cet axe. Lorsque la direction de saut est inclinée par rapport à l'axe optique, les limites entre les zones actives de la première surface peuvent être situées au droit - ou en vis-à-vis - de celles de l'autre surface, de préférence, selon cette direction de saut.

La lentille de l'invention est telle que les première et seconde surfaces comprennent en outre des zones de séparation, qui sont agencées de sorte que chaque surface soit constituée d'une alternance entre les zones actives et les zones de séparation. De plus, chaque zone active de l'une des première et seconde surfaces est au droit - ou en vis-à-vis - d'une zone de séparation de l'autre surface. Autrement dit, les deux surfaces sont partitionnées de façon complémentaire en zones actives et zones de séparation. Selon cette partition, les zones actives peuvent contribuer à la puissance optique de la lentille dans une mesure supérieure à celle des zones de séparation.

Selon une caractéristique additionnelle d'une lentille de l'invention, la forme de l'une au moins des première et seconde surfaces dans l'une au moins des zones de séparation est adaptée pour augmenter l'un au moins des angles suivants :
- un premier angle qui est formé par cette surface dans la zone de séparation avec une direction d'un saut de hauteur sagittale situé à la limite entre cette zone de séparation et une zone active adjacente avec celle-ci,
- un deuxième angle qui est formé par cette surface à la limite entre la zone de séparation et une zone active adjacente avec celle-ci, et
- un troisième angle qui est formé par l'autre surface à la limite de la zone active de cette autre surface qui est située au droit de la zone de séparation considérée, ce troisième angle étant formé par l'autre surface dans la zone active avec une direction d'un saut de hauteur sagittale situé à la limite de cette zone active.

Chacune de ces augmentations angulaires est considérée par rapport à un angle qui est situé à un même endroit de la lentille et formé par une surface fictive obtenue par soustraction des hauteurs sagittales respectives des deux surfaces, cette surface fictive présentant ainsi des sauts de hauteur sagittale qui sont situés au droit des limites entre les zones de séparation et les zones actives, et l'angle qui est formé par la surface fictive étant mesuré entre cette surface fictive et la direction de saut de hauteur sagittale, et étant aigu. Le plus souvent, la surface fictive qui est obtenue par la soustraction des hauteurs sagittales des surfaces optiques réelles est une surface de Fresnel traditionnelle, c'est-à-dire telle que connue avant la présente invention.

Selon encore une autre caractéristique d'une lentille qui est conforme à l'invention, une amplitude de variation de hauteur sagittale de chacune des première et seconde surfaces dans l'une au moins des zones de séparation de cette surface est inférieure à 25% d'une amplitude de variation de hauteur sagittale de l'autre surface dans la zone active qui est située au droit de la zone de séparation considérée.

Ces augmentations angulaires permettent à un outil d'usinage par enlèvement de matière qui est utilisé pour réaliser les surfaces optiques de la lentille, d'accéder jusqu'au sommet des angles des surfaces qui sont situés à l'endroit des sauts de hauteur sagittale.

De plus, l'une ou les deux surfaces optiques dans ses (leurs) zones de séparation peut (peuvent) avoir des formes qui sont conçues pour compenser réciproquement les écarts d'usinage qui existent dans les zones actives, entre les formes des surfaces qui sont réellement obtenues et les formes correspondantes qui sont voulues.

De cette façon, la répartition de la puissance optique de la lentille entre deux surfaces optiques qui sont distinctes, avec des partitions complémentaires de chacune de ces surfaces en zones actives et zones de séparation, permet d'améliorer la qualité optique de la lentille qui est produite.

Selon un perfectionnement de l'invention, pour l'une au moins des première et seconde surfaces de la lentille, cette surface peut présenter une forme sur un côté au moins d'un saut de hauteur sagittale de cette surface, à la limite entre une zone de séparation et une zone active, qui est adaptée pour compenser un défaut de formage au sommet d'un angle de l'autre surface, situé au droit de la limite de zones considérée. Eventuellement, une telle adaptation de forme de la surface peut être mise en oeuvre à la fois des deux côtés du saut de hauteur sagittale. Les surfaces optiques peuvent ainsi être réalisées avec une exactitude améliorée, par rapport à la surface-cible.

Dans des modes particuliers de réalisation de l'invention, un ou plusieurs des perfectionnements suivants peuvent être utilisés avantageusement, isolément ou en combinaison de plusieurs d'entre eux :
- chacune des première et seconde surfaces peut être plane ou, plus généralement, correspondre à la base plane ou cambrée de la lentille, à l'intérieur de chaque zone de séparation ;
- les zones actives et les zones de séparation peuvent être concentriques à l'intérieur de chacune des première et seconde surfaces ; et
- la partie de la puissance optique de la lentille qui est produite par les première et seconde surfaces résulte d'une réfraction ou d'une diffraction lumineuse dans les zones actives et les zones de séparation de chaque surface. Autrement dit, la lentille de l'invention peut être du type lentille de Fresnel ou lentille diffractive.

L'invention peut être appliquée avantageusement au domaine ophtalmique, pour lequel l'exigence de qualité optique est importante. Dans ce cas, la lentille est de type lentille ophtalmique, y compris verre de lunettes, lentille de contact et implant oculaire. Une telle lentille ophtalmique possède des caractéristiques supplémentaires spécifiques, telles qu'une valeur de base qui peut être ajoutée aux courbures des surfaces optiques.

Selon une constitution particulière de la lentille, celle-ci peut comprendre au moins deux parties de lentille qui sont séparées, avec une première de ces parties de lentille qui forme la première surface optique et une seconde de ces parties de lentille qui forme la seconde surface optique. Ces deux parties de lentille sont avantageusement agencées pour que les deux surfaces optiques soient tournées l'une vers l'autre. Une telle constitution est avantageuse car les deux surfaces optiques sont protégées mutuellement par rapport à des agressions ou des salissures qui proviendraient du milieu extérieur. Selon un perfectionnement d'une telle constitution, les deux parties de lentille peuvent présenter des motifs qui sont complémentaires dans une zone périphérique de la lentille, ces motifs étant adaptés pour produire un alignement des deux parties de lentille l'une par rapport à l'autre lors d'un assemblage de celles-ci pour constituer la lentille.

L'invention propose aussi un ensemble de formage d'une lentille, qui comprend deux surfaces masters-adaptés pour former respectivement les première et seconde surfaces optiques d'une lentille conforme à l'invention, telle que décrite précédemment. En particulier, cet ensemble de formage peut constituer une paire de matrices d'embossage ou une paire d'inserts de moulage. Ces matrices d'embossage ou inserts de moulage comportent alors respectivement le premier et le second surface-master.

D'autres particularités et avantages de la présente invention apparaîtront dans la description ci-après d'exemples de réalisation non limitatifs, en référence aux dessins annexés, dans lesquels :
- la figure 1 illustre une difficulté d'usinage d'un insert de moulage d'une lentille de Fresnel, telle qu'existant avant la présente invention ;
- la figure 2a est une section en coupe d'une lentille de Fresnel illustrant un premier aspect de l'invention ;
- la figure 2b est une section en coupe d'un ensemble d'inserts de moulage adapté pour produire une lentille de Fresnel conforme à la figure 2a ;
- la figure 3a est une section en coupe d'inserts de moulage selon une première mise en oeuvre de l'invention ;
- les figures 3b et 3c correspondent à la figure 3a pour deux autres mise en oeuvre de l'invention ;
- les figures 4a et 4b correspondent à la figure 3b, en utilisant un perfectionnement de l'invention ;
- les figures 5a-5c et 6a-6b correspondent respectivement aux figures 3a-3c et 4a-4b, pour un usinage direct de la lentille ;
- la figure 7 correspond à la figure 2a, pour une variante de l'invention ;
- la figure 8 correspond à la figure 2a, pour une lentille ophtalmique destinée à être assemblée dans une monture de lunettes ;
- les figures 9a à 9d correspondent à la figure 2b pour plusieurs variantes de directions de sauts de hauteur sagittale ; et
- la figure 10 est une section en coupe d'une autre lentille conforme à l'invention.

Pour raison de clarté, les dimensions des éléments qui sont représentés dans ces figures ne correspondent ni à des dimensions réelles ni à des rapports de dimensions réels. De plus, des motifs aux fonctions identiques qui existent à répétition dans une lentille ou un ensemble de formage ne sont représentés qu'un nombre restreint de fois, sans liaison avec la réalité, pour permettre leur grossissement dans les figures. Enfin, des références identiques qui sont indiquées dans des figures différentes désignent des éléments identiques ou qui ont des fonctions identiques.

L'invention est maintenant décrite en détail dans le cadre de la réalisation de lentilles de Fresnel. De façon connue, la puissance optique et l'astigmatisme éventuel d'une telle lentille résultent de la réfraction des rayons lumineux à travers les deux surfaces optiques de la lentille. Toutefois, il est entendu que la description peut être transposée à la réalisation de lentilles diffractives, en y apportant des adaptations qui sont à la portée de l'Homme du métier, sans nécessiter aucune activité inventive.

L'invention est aussi décrite dans le cas l'une lentille monolithique pour raison de simplicité, mais on indiquera à la fin de la description que la lentille peut être constituée de plusieurs parties assemblées, avec les surfaces optiques auxquelles l'invention est appliquée qui sont portées par des parties de lentille séparées.

Conformément à la figure 2a, une lentille de Fresnel 1 qui est conforme à l'invention possède au moins deux surfaces optiques S₁ et S₂. Ces surfaces sont décalées parallèlement à l'axe optique A-A de la lentille 1, de sorte qu'elles sont traversées l'une après l'autre par des mêmes rayons lumineux qui forment une image à travers la lentille. Par exemple, S₁ et S₂ peuvent être respectivement la surface optique antérieure et la surface optique postérieure de la lentille 1, par rapport au sens de propagation des rayons lumineux.

De façon connue, chaque surface S₁, S₂ peut être repérée par des valeurs de hauteur sagittale qui sont définies pour tous les points de cette surface. D'une façon simple, la hauteur sagittale d'un point de chaque surface S₁, S₂ est la distance, mesurée parallèlement à l'axe optique A-A, entre cette surface et un plan de référence qui est perpendiculaire à l'axe A-A. Sur la figure 2a, l'axe de mesure de hauteur sagittale est noté z, parallèle à l'axe optique A-A, et z₁ et z₂ sont les valeurs respectives de hauteur sagittale des surfaces S₁ et S₂. La hauteur sagittale z₁, z₂ de chaque surface S₁, S₂ présente alors des variations continues à l'intérieur de zones dans lesquelles cette surface est elle-même continue. Par contre, entre deux zones adjacentes, chaque surface peut présenter une discontinuité de hauteur sagittale le long d'une limite intermédiaire entre ces zones, avec une valeur de saut de hauteur sagittale qui constitue une mesure de la discontinuité. Chaque surface peut aussi être continue le long d'une limite intermédiaire entre deux zones, mais présenter une rupture de plan tangent, ou discontinuité d'inclinaison, le long de cette limite.

Selon une première caractéristique de l'invention, les deux surfaces optiques S₁ et S₂ sont chacune partitionnées selon un découpage commun. Lorsque les zones de découpage des deux surfaces sont concentriques, l'usinage de la lentille ou d'un élément de formage de celle-ci est simplifié, et peut être réalisé avec un tour. Ainsi, la surface S₁ est divisée selon deux familles de zones qui sont alternées, respectivement des zones actives qui sont désignées par ZA₁ et des zones de séparation qui sont désignées par ZS₁. De la même façon, la surface S₂ est simultanément divisée en zones actives ZA₂ qui sont alternées avec des zones de séparation ZS₂. Les limites entre zones adjacentes ZA₁/ZS₁ dans la surface S₁ sont alignées selon l'axe optique A-A, avec les limites entre zones adjacentes ZA₂/ZS₂ dans la surface S₂. En ce sens, les limites entre les zones ZA₁/ZS₁ sont en vis-à-vis des limites entre les zones ZA₂/ZS₂. De plus, chaque zone active ZA₁ de la surface S₁ est située au droit selon l'axe A-A, ou en vis-à-vis, d'une zone de séparation ZS₂ de la surface S₂. Simultanément, chaque zone de séparation ZS₁ est située au droit de d'une zone active ZA₂.

Comme expliqué plus loin, les zones actives des deux surfaces optiques produisent une partie essentielle, voir la totalité de la puissance optique et de l'astigmatisme éventuel de la lentille 1. Pour cela, chaque surface S₁, S₂ possède des valeurs de courbures appropriées dans ses zones actives, ZA₁, ZA₂. A titre d'exemple, les surfaces optiques S₁ et S₂ qui sont représentées dans les figures correspondent à des lentilles divergentes, car ces surfaces sont concaves pour la lentille dans les zones actives ZA₁ et ZA₂. La contribution, dite de Fresnel, de chaque surface optique S₁, S₂ dans les zones de séparation correspondantes ZS₁, ZS₂, à la puissance optique et à l'astigmatisme éventuel de la lentille 1, est de préférence soit nulle, soit inférieure à celle des zones actives ZA₁, ZA₂. En effet, cette surface est confondue avec la base, ou proche de cette base, dans les zones de séparation, que la base soit plane ou cambrée. En fait, comme cela sera expliqué plus loin, lorsqu'elles ne sont pas nulles, les contributions des surfaces S₁, S₂ dans les zones de séparation ZS₁, ZS₂ peuvent être réduites à une fonction de compensation de défauts des contributions des zones actives ZA₁, ZA₂, par rapport à une surface-cible de la lentille 1 qui correspond à ses valeurs de puissance optique et d'astigmatisme. Lorsque les contributions des zones de séparation ZS₁, ZS₂ à la puissance optique et à l'astigmatisme de la lentille sont nulles, les surfaces S₁ et S₂ peuvent être planes ou, plus généralement, être confondues avec la base à l'intérieur de ces zones.

De façon connue, on entend par surface équivalente à une lentille à deux surfaces optiques, la surface fictive S_{F} qui est définie en soustrayant ses surfaces optiques : z_{F} = z₁ - z₂, où z₁ est la hauteur sagittale de la surface S₁, z₂ est la hauteur sagittale de la surface S₂, et z_{F} est la hauteur sagittale de la surface fictive S_{F}, les trois hauteurs sagittales z₁, z₂ et z_{F} étant considérées pour l'opération de soustraction de surfaces, en des points qui sont alignés parallèlement à l'axe optique A-A. Dans cette opération de soustraction de surfaces qui est réalisée point-à-point selon une direction fixe, le signe moins correspond à une orientation algébrique des surfaces S₁ et S₂ par rapport au sens de propagation des rayons lumineux à travers la lentille 1. Dans le bas de la figure 2a, la surface S_{F} qui est représentée est la surface fictive qui est équivalente à la lentille de Fresnel 1 de la même figure.

La fonction optique d'une lentille est fixée par une surface-cible qui est adoptée pour la surface fictive S_{F} équivalente à la lentille. Or une même surface-cible peut être réalisée de différentes façons en associant des surfaces optiques S₁ et S₂ qui sont appropriées pour reproduire la surface fictive-cible en étant soustraites l'une à l'autre. La caractéristique de l'invention qui procure aux zones de séparation ZS₁, ZS₂ de chaque surface S₁, S₂ une fonction de compensation par rapport aux zones actives, est basée sur ce principe.

La figure 2b représente une paire d'inserts de moulage qui est adaptée pour produire la lentille 1 de la figure 2a. Les deux inserts sont désignés respectivement par les références 2 et 3, et sont destinés à être placés en vis-à-vis à l'intérieur d'un moule d'injection du polymère de la lentille 1. Par conséquent, les inserts 2 et 3 possèdent des surfaces-masters qui correspondent aux surfaces S₁ et S₂ de la lentille 1 à produire. Pour cette raison, les surfaces S₁ et S₂ sont reportées sur la figure 2b, avec les zones ZA₁, ZS₁, ZA₂ et ZS₂. La description présente est d'abord poursuivie dans le cas particulier des inserts de moulage, mais il est entendu qu'elle peut être transposée directement à des matrices d'embossage.

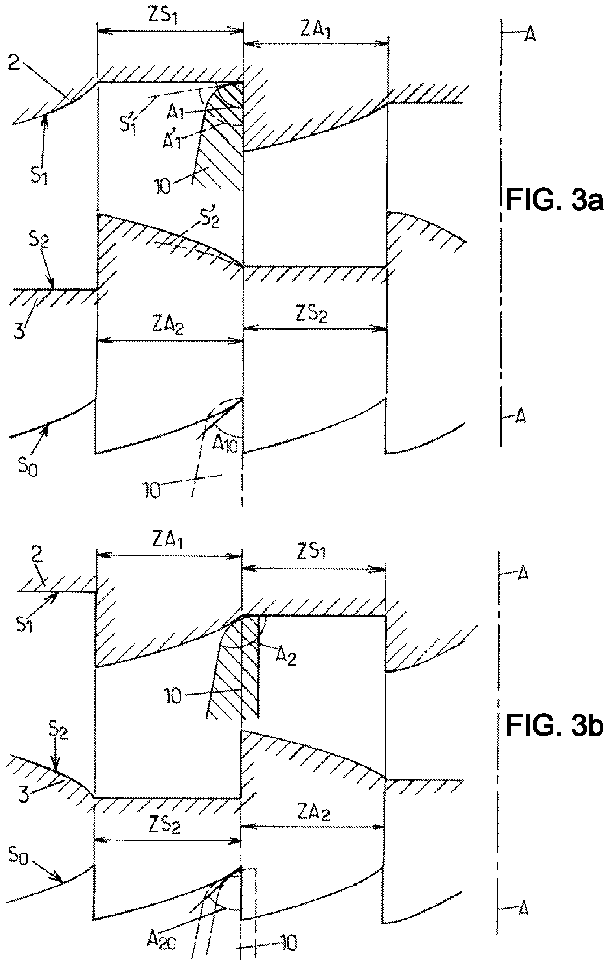

Les figures 3a-3c qui sont maintenant décrites successivement, correspondent à trois mises en oeuvre séparées de l'invention pour les inserts de moulage 2 et 3. Chacune de ces figures est une vue grossie des surfaces S₁ et S₂, en reprenant leur alignement selon l'axe A-A. Elle montre aussi la surface-cible S₀ correspondante, pour faire apparaître le gain qui est apporté par l'invention. La direction des sauts de hauteur sagittale est encore l'axe optique A-A.

Ces figures montrent des modifications d'angles formés par l'une ou l'autre des surfaces S₁ et S₂, qui sont obtenues grâce à l'adaptation de la surface S₁ dans une zone de séparation ZS₁ conformément à l'invention. Mais il est entendu qu'un avantage identique peut être obtenu en appliquant l'invention à la surface S₂. En outre, les modes de réalisation qui sont montrés peuvent être combinés entre eux, pour obtenir des combinaisons de leurs avantages respectifs.

La référence 10 désigne l'outil d'usinage à enlèvement de matière qui est utilisé pour réaliser les inserts 2 et 3 en utilisant un tour. Une position de cet outil d'usinage est indiquée sur chacune des figures 3a-3c, qui est permise par l'invention sur l'une ou l'autre des surfaces S₁ ou S₂, et dont la position d'outil correspondante n'est pas possible pour la surface-cible S₀ si cette dernière était adoptée pour former une seule surface optique de lentille.

La mise en oeuvre de l'invention qui est illustrée par la figure 3a est la plus simple. Elle concerne le placement de l'outil d'usinage 10 pour former le fond d'un saut de hauteur sagittale, lorsque la surface d'insert S₁ est en creux dans les zones de séparation ZS₁ par rapport aux zones actives adjacentes ZA₁. Dans un premier temps, on suppose que chacune des surfaces S₁, S₂ est plane dans les zones de séparation correspondantes. La surface S₁ forme donc un angle A₁ qui est égal à 90° entre cette surface dans la zone de séparation ZS₁ et la direction du saut de hauteur sagittale qui sépare cette zone ZS₁ de la zone active ZA₁ adjacente. L'angle correspondant A₁₀ sur la surface-cible S₀ est quant à lui inférieur à 90°, du fait de l'inclinaison de la surface S₂ dans la zone active ZA₂ qui est au droit de la zone ZS₁. L'introduction de l'outil 10 jusqu'à la pointe de l'angle A₁ est possible sur la surface S₁, alors qu'il ne serait pas possible d'introduire le même outil 10 jusqu'à la pointe de l'angle A₁₀ sur la surface-cible S₀ (voir la position d'outil indiquée en traits interrompus).

Les deux lignes interrompues S'₁ et S'₂ montrent des formes alternatives possibles pour les surfaces S₁ et S₂, respectivement dans les zones ZS₁ et ZA₂, qui correspondent à la même surface-cible S₀. Le nouvel angle A'₁ est encore supérieur à l'angle A₁₀, et la surface S'₁ pourrait encore être réalisée avec l'outil 10 d'une façon qui serait améliorée par rapport à un usinage de la surface-cible S₀.

La mise en oeuvre de l'invention de la figure 3b concerne le placement de l'outil d'usinage 10 pour réaliser la surface S₁ autour d'une limite entre deux zones ZA₁ et ZS₁ qui sont adjacentes, lorsque la surface d'insert S₁ est encore en creux dans les zones ZS₁ par rapport aux zones ZA₁, et lorsque la surface S₁ est continue entre les deux zones ZA₁ et ZS₁ qui sont considérées. Au niveau de cette limite de zones, la surface S₁ forme un angle A₂ entre ses portions qui sont respectivement dans la zone de séparation ZS₁ et dans la zone active ZA₁. Cet angle A₂ est obtus, c'est-à-dire supérieur à 90°, alors que l'angle A₂₀ de la surface-cible S₀ est aigu à cause du saut de hauteur sagittale qui est réalisé par la surface S₂ à cet endroit. Les positions indiquées pour l'outil 10 montrent que celui-ci pénètre dans l'angle A₂ plus loin que cela aurait été possible dans l'angle A₂₀ de la surface-cible S₀.

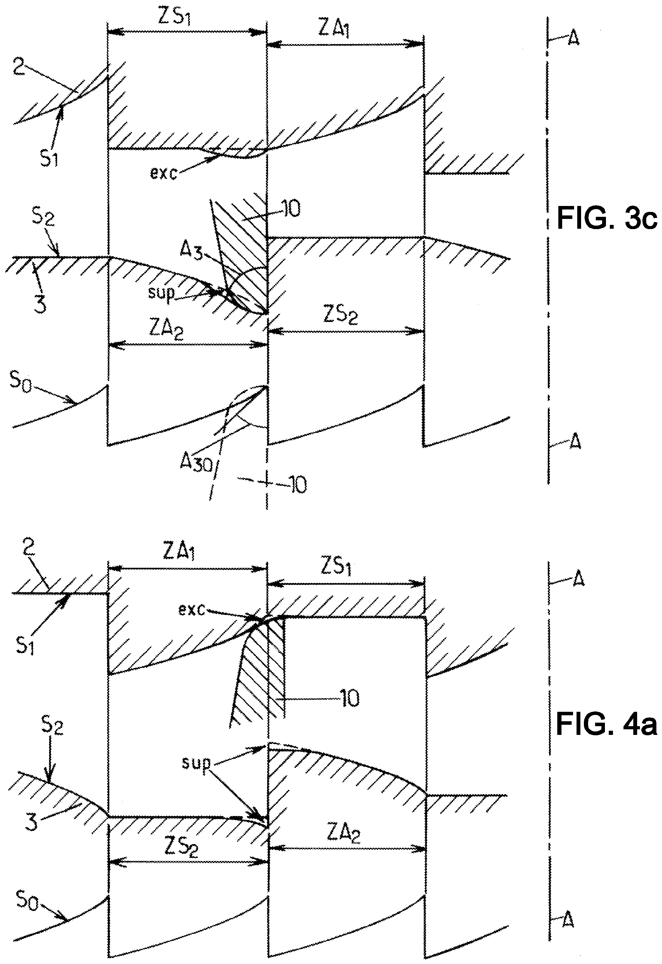

Enfin, la mise en oeuvre de la figure 3c concerne une autre configuration, dans laquelle les surfaces d'insert S₁ et S₂ sont en creux dans les zones actives ZA₁, respectivement ZA₂, par rapport aux zones de séparation ZS₁, respectivement ZS₂. L'angle A₃ est l'angle rentrant aigu qui est formé par la surface S₂ à l'endroit d'un saut de hauteur sagittale, entre la direction du saut et la portion de la surface S₂ qui est située dans la zone active ZA₂. Cet angle peut être augmenté en retirant un supplément de matière de l'insert 3 dans la zone ZA₂, désigné par sup, et en compensant ce supplément de matière retirée de l'insert 3 par un excédent de matière exc qui est laissé volontairement sur la surface S₁ dans la zone ZS₁. Le profil d'épaisseur du supplément de matière retirée sup de l'insert 3 peut avantageusement correspondre au profil de l'outil 10, pour que ce dernier puisse pénétrer jusqu'au sommet de l'angle A₃. Le profil d'épaisseur de l'excédent de matière exc qui est laissé sur la surface S₁ correspond de préférence à celui du supplément de matière retirée sup, pour que la surface fictive résultante coïncide encore avec la surface-cible S₀. L'angle A₃ de la surface S₂ qui est ainsi modifiée devient plus grand que l'angle A₃₀ de la surface-cible S₀.

Grâce à l'amélioration de la fidélité d'usinage qui est ainsi obtenue pour ces trois configurations, par rapport à la surface-cible S₀, la lentille 1 possède une fonction optique qui est plus proche de la fonction optique de cible. En particulier, la lentille 1 ainsi conçue selon l'invention produit moins de lumière diffusée que la lentille équivalente dont une seule face comporterait des zones de Fresnel adjacentes, sans zones de séparation intermédiaires, pour des surfaces-cibles S₀ identiques. Typiquement, une réduction de largeur dans un facteur quatre, pour les bandes dans lesquelles la surface fictive S_{F} qui est équivalente à la lentille réelle 1 ne correspond pas à la surface-cible S₀, produit une variation de la valeur 88% environ à la valeur 97% environ, pour le contraste de formation d'image à travers la lentille.

La figure 4a illustre un perfectionnement de l'invention, dans le cas particulier de la configuration qui a déjà été envisagée à la figure 3b. Malgré l'augmentation de l'angle A₂ qui a été obtenue par l'invention, il est possible que l'outil 10 ne puisse encore pas former la pointe extrême de l'angle A₂, notamment lorsque l'outil 10 présente un dos arrondi du côté opposé à son angle d'attaque. Dans ce cas, l'usinage laisse un excès de matière sur la surface S₁ de l'insert 2, à la pointe de l'angle A₂. Cet excès peut alors être compensé en retirant un supplément de matière sur la surface S₂ de l'insert 3, au droit de l'angle A₂. En fait, ce perfectionnement combine l'effet de l'invention qui vient d'être présenté pour l'angle A₃ (figure 3c), avec l'invention appliquée pour l'angle A₂. Pour cette raison, l'excès de matière qui est laissé sur la surface S₁ de l'insert 2 est aussi indiqué exc sur la figure 4a, et le supplément de matière qui est retiré à la surface S₂ de l'insert 3 est indiqué sup. La surface-cible S₀ peut ainsi être obtenue avec une exactitude supérieure au niveau des limites entre zones actives et zones de séparation auxquelles ce perfectionnement est appliqué.

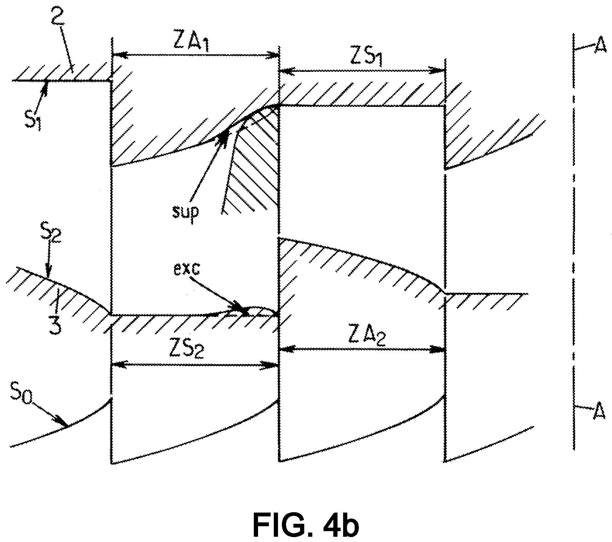

Sur la figure 4a, la surface S₁ de l'insert 2 a été usinée de sorte que l'excédent de matière exc soit laissé de part et d'autre de la limite entre les zones ZA₁ et ZS₁. La figure 4b montre le cas où la surface S₁ est réalisée avec une trajectoire de l'angle d'attaque de l'outil 10 qui passe par le sommet de l'angle A₂. Un supplément de matière sup est alors retiré de l'insert 2, dans la zone active ZA₁ uniquement. L'excédent de matière exc qui est à laisser sur la surface S₂ de l'insert 3 en compensation, est alors limité à l'intérieur de la zone de séparation ZS₂.

Par ailleurs, chaque surface S₁ ou S₂ peut être réalisée en effectuant un déplacement de l'outil 10 selon une seule direction, avec son angle d'attaque qui est orienté vers l'avant du déplacement. Cette direction de déplacement peut être radiale, centrifuge ou centripète, par rapport à la lentille 1 lorsqu'un tour est utilisé. L'usinage de chaque surface S₁, S₂ peut aussi être effectué en deux passages de l'outil 10, avec chaque passage dans un sens de déplacement de l'outil 10 qui est opposé au sens de déplacement de l'autre passage, et en retournant l'outil 10 lors d'une inversion du sens de passage.

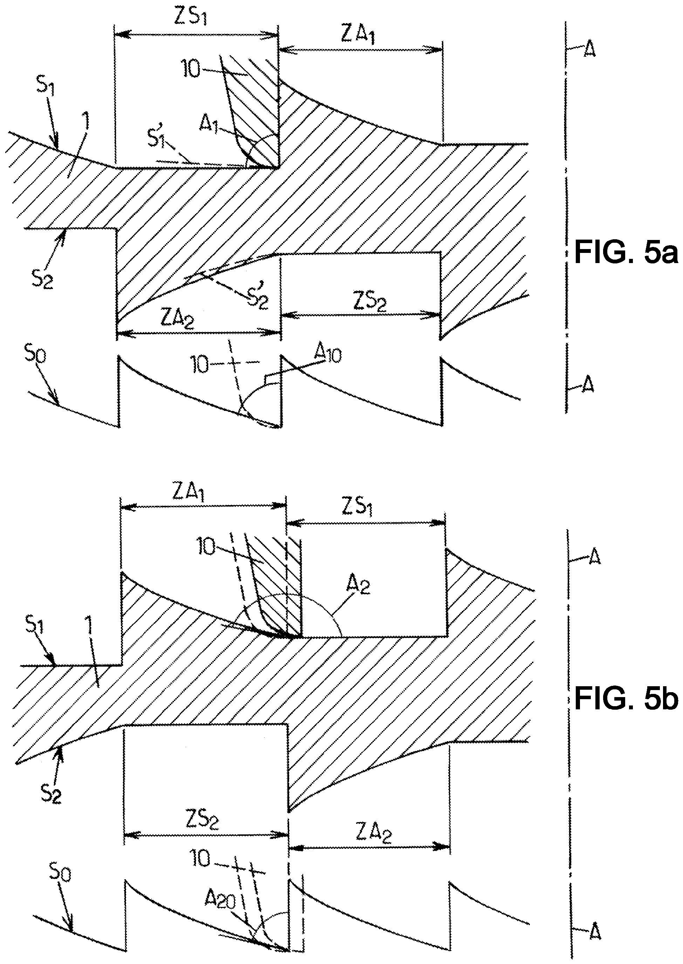

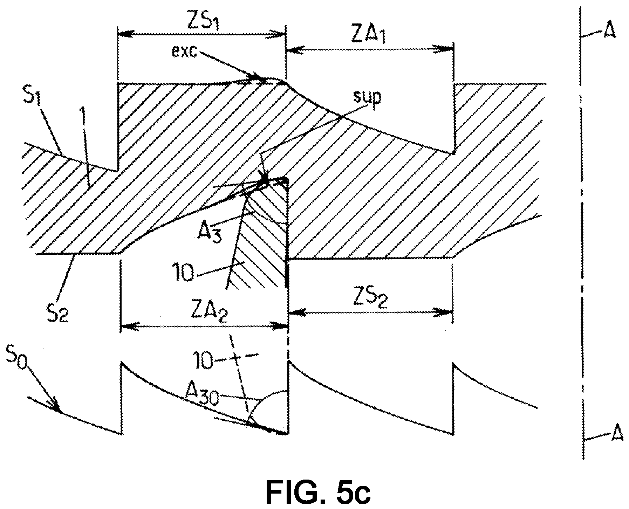

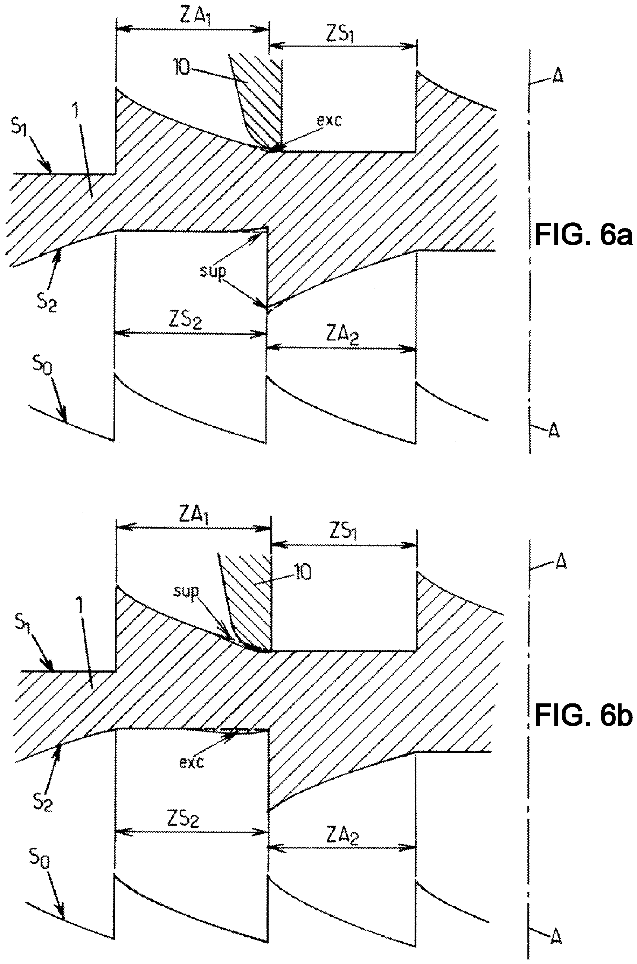

Bien que l'invention vienne d'être décrite dans le cas de l'usinage des inserts de moulage 2 et 3, cette description peut être transposée au cas d'un usinage direct de la lentille 1. Les figures 5a-5c et 6a-6b illustrent les gains qui sont procurés par l'invention pour un tel usinage direct. Les angles qui sont considérés sont similaires à ceux des figures 3a-3c et 4a-4b, si bien qu'il n'est pas nécessaire de répéter les explications. Les positions de l'outil 10 sont seulement inversées par rapport aux surfaces optiques S₁ et S₂, du fait de l'usinage direct de la lentille 1 : l'outil 10 est maintenant à l'extérieur de l'intervalle qui est compris entre ces surfaces S₁ et S₂.

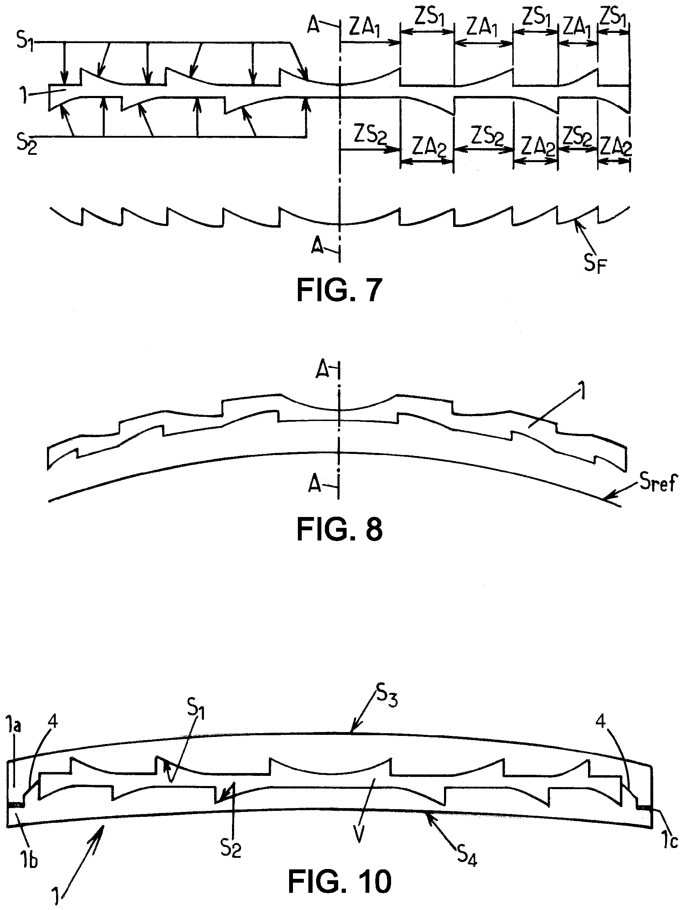

Toutefois, des configurations sont préférées pour la lentille 1, dans lesquelles une des surfaces optiques S₁ et S₂, ou les deux, est (sont) en creux dans l'une au moins des zones de séparation, ZS₁ ou ZS₂ respectivement, par rapport à cette (ces) même(s) surface(s) dans les zones actives, ZA₁ ou ZA₂ respectivement, qui sont adjacentes à la zone de séparation considérée. Le poids de la lentille 1 peut alors être réduit, pour une fonction optique de la lentille qui est identique. D'une façon encore plus préférée, chacune des surfaces optiques S₁ et S₂ de la lentille 1 peut être en creux dans chaque zone de séparation par rapport à la même surface dans les zones actives qui sont adjacentes à la zone de séparation considérée. La figure 7 correspond à la figure 2a pour une telle configuration des surfaces optiques de la lentille.

Il est entendu que l'invention qui vient d'être décrite en détail pour une lentille de Fresnel divergente peut être appliquée de la même façon à une lentille de Fresnel convergente.

Il est aussi entendu que l'invention peut être appliquée de la même façon à une lentille ophtalmique comme représenté sur la figure 8. Pour cela, une valeur non nulle de courbure de référence, appelée courbure de base, est ajoutée aux deux surfaces optiques S₁ et S₂. Une telle valeur de base peut notamment être intégrée à la surface de référence qui est utilisée pour mesurer les hauteurs sagittales des deux surfaces optiques. La surface de référence est alors couramment appelée base dans le jargon de l'Homme du métier. Sur la figure 8, S_{ref} désigne une telle base, ou surface de référence à courbure non nulle.

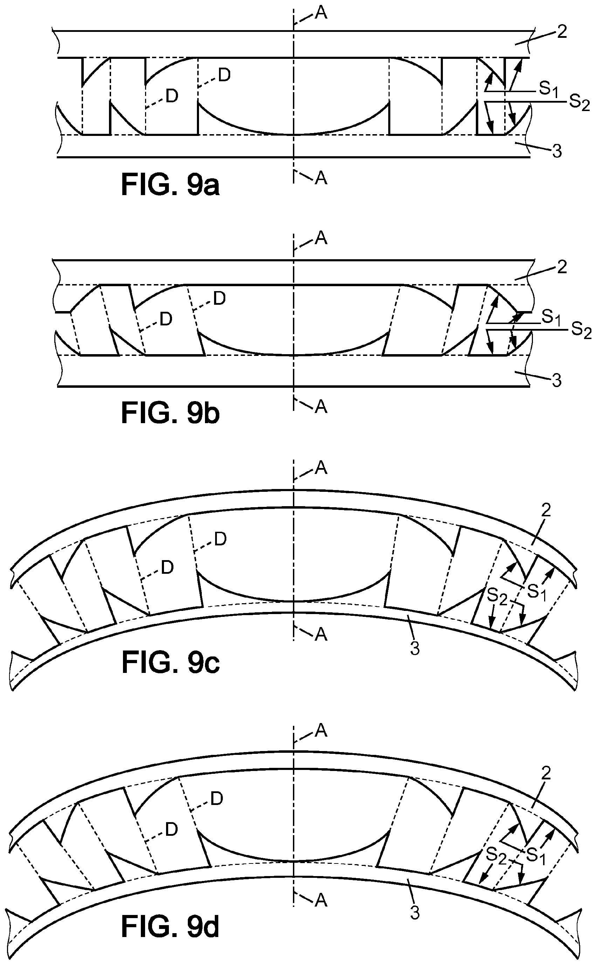

Les figures 9a et 9b illustrent des réalisations de l'invention lorsque la courbure de référence est nulle, autrement dit lorsque la base de la lentille est plane, et les figures 9c et 9d d'autres réalisations de l'invention lorsque la base est cambrée. Dans les réalisations des figures 9a et 9c, les directions D d'alignement des sauts de hauteur qui sont situés en vis-à-vis, respectivement sur les surfaces S₁ et S₂, sont perpendiculaires à la base, qui est représentée en traits interrompus au niveau de chaque surface. Du fait de la base plane pour la figure 9a, la direction D est parallèle à l'axe optique A-A de la lentille. En outre, la direction D est la direction de chaque saut de hauteur, c'est-à-dire la direction de discontinuité de chacune des surfaces S₁ et S₂. Toutefois, d'une façon générale, la direction des sauts de hauteurs peut être inclinée par rapport à la direction qui est perpendiculaire à la base à l'endroit du saut de hauteur. Une telle possibilité est représentée sur la figure 9b pour la base plane, et sur la figure 9d pour une base cambrée. Dans les deux cas, la direction des sauts de hauteur est encore confondue avec la direction D d'alignement des limites de zones qui sont situées en vis-à-vis. Une telle configuration est préférée, car elle diminue la visibilité de la structure à zones de la lentille, ainsi que la gêne qui pourrait en résulter pour un utilisateur de celle-ci.

Il est encore entendu que l'invention peut être appliquée à des lentilles chacune à plus de deux surfaces optiques, par exemple trois ou quatre surfaces optiques, qui sont traversées successivement par les rayons lumineux. Dans ce cas, les zones actives et les zones de séparation de toutes ces surfaces optiques peuvent être combinées de différentes façons, tout en appliquant les caractéristiques de l'invention. A partir de la description qui a été fournie de la présente invention, l'Homme du métier saura appliquer sans difficulté l'invention à de telles lentilles plus complexes. De plus, les surfaces optiques auxquelles l'invention est appliquée peuvent être exposées au milieu ambiant, ou être recouvertes d'une couche transparente, par exemple d'une couche de résine. Une telle couche, qui possède une valeur d'indice de réfraction lumineuse différente de celle du matériau polymère de la lentille, peut notamment protéger la structure à sauts de hauteur sagittale contre des rayures ou des dépôts de salissures.

Il est aussi possible de constituer une lentille selon l'invention, en réalisant séparément plusieurs parties de lentille qui sont ensuite assemblées les unes avec les autres pour constituer la lentille complète. Dans ce cas, les surfaces optiques auxquelles l'invention est appliquée peuvent être portées par des parties différentes de la lentille. Leur assemblage est alors effectué de façon à aligner les limites entre zones actives et zones de séparation des parties séparées initialement, selon l'axe optique de la lentille, pour obtenir les avantages de l'invention. Un tel assemblage peut être effectué par collage des parties les unes aux autres, par exemple en utilisant un collage qui est limité à un contour périphérique de la lentille.

La figure 10 est une vue en coupe d'une telle lentille 1 qui est conforme à l'invention, en étant constituée de deux parties de lentilles 1a et 1b conçues pour que les surfaces optiques zonées S₁ et S₂ soient tournées vers l'intérieur de la lentille. La référence 1c indique la zone périphérique de collage des parties 1a et 1b entre elles. La référence 4 désigne des motifs en relief qui sont portés par les deux parties de lentilles 1a et 1b à l'extérieur de leurs surfaces optiques respectives S₁ et S₂, par exemple autour de ces surfaces. Ces motifs 4 sont complémentaires d'une partie de lentille à l'autre, de sorte que leur emboîtement garantisse l'alignement des surfaces optiques S₁ et S₂ qui est prévu par l'invention. Eventuellement, l'espace V qui est enfermé entre les surfaces S₁ et S₂ peut être rempli d'une façon adaptée pour conférer à la lentille 1 une fonction supplémentaire. Les surfaces optiques extérieures de la lentille 1 qui est ainsi constituée, référencées S₃ et S₄, peuvent être usinées en outre pour conférer des contributions complémentaires à la puissance optique totale de la lentille. Un tel usinage de l'une ou des deux surfaces S₃ et S₄ est de préférence réalisé après l'assemblage définitif des deux parties de lentille 1a et 1b.

## Revendications

1. Lentille (1) comprenant au moins deux surfaces optiques (S₁, S₂) s'étendant chacune transversalement par rapport à un axe optique (A-A) de la lentille, de sorte que des rayons lumineux qui traversent la lentille traversent l'une puis l'autre des deux surfaces, chaque surface comprenant des zones actives (ZA₁, ZA₂) à l'intérieur desquelles une forme de ladite surface est adaptée pour produire une partie au moins de la puissance optique de la lentille pour les rayons qui traversent les dites zones actives, avec une discontinuité de hauteur sagittale de chaque surface entre deux zones actives différentes de ladite surface,
des limites des zones actives (ZA₁, ZA₂) d'une première des deux surfaces étant situées au droit de limites des zones actives de l'autre des dites deux surfaces,
les dites première et seconde surfaces (S₁, S₂) comprenant en outre des zones de séparation (ZS₁, ZS₂) agencées de sorte que chacune des première et seconde surfaces soit constituée d'une alternance entre les zones actives et les zones de séparation,
chaque zone active (ZA₁, ZA₂) de l'une des première et seconde surfaces (S₁, S₂) étant au droit d'une zone de séparation (ZS₂, ZS₁) de l'autre des dites première et seconde surfaces, et
la forme de l'une au moins des première et seconde surfaces (S₁, S₂) dans l'une au moins des zones de séparation (ZS₁, ZS₂) est adaptée pour augmenter l'un au moins des angles suivants :
- un premier angle (A₁) formé par ladite surface (S₁, S₂) dans ladite zone de séparation (ZS₁, ZS₂) avec une direction d'un saut de hauteur sagittale situé à une limite entre ladite zone de séparation et une zone active (ZA₁, ZA₂) adjacente avec ladite zone de séparation,
- un deuxième angle (A₂) formé par ladite surface (S₁, S₂) à une limite entre ladite zone de séparation (ZS₁, ZS₂) et une zone active (ZA₁, ZA₂) adjacente avec ladite zone de séparation, et
- un troisième angle (A₃) formé par l'autre surface (S₂, S₁) à une limite de la zone active (ZA₂, ZA₁) de ladite autre surface qui est située au droit de ladite zone de séparation (ZS₁, ZS₂), ledit troisième angle étant formé par ladite autre surface dans ladite zone active avec une direction d'un saut de hauteur sagittale situé à la limite de ladite zone active,
par rapport à un angle (A₁₀, A₂₀, A₃₀) situé à un même endroit de la lentille (1) et formé par une surface fictive (S₀) obtenue par soustraction des hauteurs sagittales respectives des première et seconde surfaces (S₁, S₂), ladite surface fictive présentant ainsi des sauts de hauteur sagittale situés au droit des limites entre les zones de séparation et les zones actives, et l'angle (A₁₀, A₂₀, A₃₀) formé par la surface fictive étant mesuré entre ladite surface fictive et la direction de saut de hauteur sagittale, et étant aigu,
la lentille étant **caractérisée en ce qu'**une amplitude de variation de hauteur sagittale de chacune des première et seconde surfaces (S₁, S₂) dans l'une au moins des zones de séparation (ZS₁, ZS₂) de ladite surface est inférieure à 25% d'une amplitude de variation de hauteur sagittale de l'autre surface dans la zone active (ZA₁, ZA₂) située au droit de ladite zone de séparation.

2. Lentille selon la revendication 1 dans laquelle, pour l'une au moins des première et seconde surfaces (S₁, S₂), ladite surface dans l'une au moins des zones de séparation (ZS₁, ZS₂) est en creux par rapport à ladite surface dans les zones actives (ZA₁, ZA₂) adjacentes à ladite zone de séparation.

3. Lentille selon la revendication 2 dans laquelle, pour chacune des première et seconde surfaces (S₁, S₂), ladite surface dans chaque zone de séparation (ZS₁, ZS₂) est en creux par rapport à ladite surface dans les zones actives (ZA₁, ZA₂) adjacentes à ladite zone de séparation.

4. Lentille selon la revendication 1 dans laquelle chacune des première et seconde surfaces (S₁, S₂) est plane ou correspond à une base cambrée de ladite lentille à l'intérieur de chaque zone de séparation (ZS₁, ZS₂).

5. Lentille selon l'une quelconque des revendications précédentes dans laquelle, pour l'une au moins des première et seconde surfaces (S₁, S₂), ladite surface présente une forme sur un côté au moins d'un saut de hauteur sagittale de ladite surface, à la limite entre une zone de séparation (ZS₁, ZS₂) et une zone active (ZA₁, ZA₂), adaptée pour compenser un défaut de formage au sommet d'un angle (A₂) de l'autre surface situé au droit de ladite limite.

6. Lentille selon l'une quelconque des revendications précédentes, dans laquelle les zones actives (ZA₁, ZA₂) et les zones de séparation (ZS₁, ZS₂) sont concentriques à l'intérieur de chacune des première et seconde surfaces (S₁, S₂).

7. Lentille selon l'une quelconque des revendications précédentes, pour laquelle la partie de la puissance optique de ladite lentille qui est produite par les première et seconde surfaces (S₁, S₂) résulte d'une réfraction ou d'une diffraction lumineuse dans les zones actives (ZA₁, ZA₂) et les zones de séparation (ZA₁, ZA₂) de chaque surface.

8. Lentille selon l'une quelconque des revendications précédentes, de type lentille ophtalmique, y compris verre de lunettes, lentille de contact et implant oculaire.

9. Lentille selon l'une quelconque des revendications précédentes, comprenant au moins deux parties de lentille (1a, 1b) séparées, une première des dites parties de lentille formant la première surface optique (S₁), et une seconde des dites parties de lentille formant la seconde surface optique (S₂), et les première et seconde parties de lentille étant agencées de sorte que les dites première et seconde surfaces optiques soient tournées l'une vers l'autre.

10. Lentille selon la revendication 9, dans laquelle les première et seconde parties de lentille (1a, 1b) présentent des motifs complémentaires (4) dans une zone périphérique de la lentille, les dits motifs étant adaptés pour produire un alignement des dites première et seconde parties de lentille l'une par rapport à l'autre lors d'un assemblage des dites parties de lentille pour constituer la lentille.

11. Ensemble de formage d'une lentille, comprenant deux surfaces-masters adaptés pour former respectivement les première et seconde surfaces optiques (S₁, S₂) d'une lentille (1) selon l'une quelconque des revendications 1 à 10.

12. Ensemble de formage selon la revendication 11, constituant une paire de matrices d'embossage ou une paire d'inserts de moulage (2, 3), lesdites matrices d'embossage ou inserts de moulage comportant respectivement le premier et le second surface-master.

## Patentansprüche

1. Linse (1) mit mindestens zwei optischen Oberflächen (S₁, S₂), die sich jeweils quer zu einer optischen Achse (A-A) der Linse derart erstrecken, dass Lichtstrahlen, die durch die Linse hindurchtreten, durch die eine und dann die andere der zwei Oberflächen hindurchtreten, wobei jede Oberfläche aktive Bereiche (ZA₁, ZA₂) aufweist, in denen eine Form der Oberfläche geeignet ist, um mindestens einen Teil der optischen Leistung der Linse für die Strahlen, die durch die aktiven Bereiche hindurchtreten, mit einer Unterbrechung der Sagittalhöhe von jeder Oberfläche zwischen zwei verschiedenen aktiven Bereichen der Oberfläche zu erzeugen,
wobei Grenzen der aktiven Bereiche (ZA₁, ZA₂) von einer ersten der zwei Oberflächen direkt an den Grenzen der aktiven Bereiche der anderen der zwei Oberflächen angeordnet sind,
wobei die erste und die zweite Oberfläche (S₁, S₂) ferner Trennbereiche (ZS₁, ZS₂) aufweisen, die derart ausgebildet sind, dass jede der ersten und der zweiten Oberfläche aus einem Wechsel zwischen den aktiven Bereichen und den Trennbereichen gebildet ist,
wobei jeder aktive Bereiche (ZA₁, ZA₂) von einer der ersten und der zweiten Oberfläche (S₁, S₂) direkt an einem Trennbereich (S₁, ZS₂) der anderen der ersten und der zweiten Oberfläche ist, und
die Form von mindestens einer der ersten und der zweiten Oberfläche (S₁, S₂) in mindestens einem der Trennbereiche (ZS₁, ZS₂) geeignet ist, um mindestens einen der folgenden Winkel zu erhöhen:
- einen ersten Winkel (A₁), der durch die Oberfläche (S₁, S₂) in dem Trennbereich (S₁, ZS₂) gebildet ist, mit einer Richtung eines Sprungs der Sagittalhöhe, der an einer Grenze zwischen dem Trennbereich und einem aktiven Bereich (ZA₁, ZA₂) angeordnet ist, der angrenzend an den Trennbereich ist,
- einen zweiten Winkel (A₂), der durch die Oberfläche (S₁, S₂) an einer Grenze zwischen dem Trennbereich (S₁, ZS₂) und einem aktiven Bereich (ZA₁, ZA₂) gebildet ist, der angrenzend an den Trennbereich ist, und
- einen dritten Winkel (A₃), der durch die andere Oberfläche (S₂, S₁) an einer Grenze des aktiven Bereichs (ZA₂, ZA₁) der anderen Oberfläche gebildet ist, die direkt an dem Trennbereich (S₁, ZS₂) angeordnet ist, wobei der dritte Winkel durch die andere Oberfläche in dem aktiven Bereich mit einer Richtung eines Sprungs der Sagittalhöhe, der an der Grenze des aktiven Bereichs angeordnet ist, gebildet ist,
bezogen auf einen Winkel (A₁₀, A₂₀, A₃₀), der an einer gleichen Stelle der Linse (1) angeordnet ist und durch eine fiktive Fläche (S₀) gebildet ist, die durch Subtrahieren der jeweiligen Sagittalhöhen von der ersten und der zweiten Fläche (S₁, S₂) erhalten wird, wobei die fiktive Fläche somit Sprünge der Sagittalhöhe aufweist, die direkt an den Grenzen zwischen den Trennbereichen und den aktiven Bereichen angeordnet sind, und wobei der Winkel (A₁₀, A₂₀, A₃₀), der von der fiktiven Fläche gebildet ist, zwischen der fiktiven Fläche und der Richtung des Sprungs der Sagittalhöhe gemessen wird und spitz ist,
wobei die Linse **dadurch gekennzeichnet ist, dass** eine Amplitude der Änderung der Sagittalhöhe von jeder der ersten und der zweiten Oberfläche (S₁, S₂) in mindestens einem der Trennbereiche (S₁, ZS₂) der Oberfläche niedriger als 25 % einer Amplitude der Änderung der Sagittalhöhe der anderen Oberfläche in dem aktiven Bereich (ZA₁, ZA₂) ist, der direkt an dem Trennbereich angeordnet ist.

2. Linse nach Anspruch 1, wobei für mindestens eine der ersten und der zweiten Oberfläche (S₁, S₂) die Oberfläche in mindestens einem der Trennbereiche (ZS₁, ZS₂) gegenüber der Oberfläche in den aktiven Bereichen (ZA₁, ZA₂), die angrenzend an dem Trennbereich sind, vertieft ist.

3. Linse nach Anspruch 2, wobei für jede der ersten und der zweiten Oberfläche (S₁, S₂) die Oberfläche in jedem der Trennbereiche (ZS₁, ZS₂) gegenüber der Oberfläche in den aktiven Bereichen (ZA₁, ZA₂), die angrenzend an dem Trennbereich sind, vertieft ist.

4. Linse nach Anspruch 1, wobei jede der ersten und der zweiten Oberfläche (S₁, S₂) eben ist oder einer gewölbten Basis der Linse im Inneren von jedem Trennbereich (S₁, ZS₂) entspricht.

5. Linse nach einem der vorhergehenden Ansprüche, wobei für mindestens eine der ersten und der zweiten Oberfläche (S₁, S₂) die Oberfläche auf mindestens einer Seite eine Form von einem Sprung der Sagittalhöhe der Oberfläche an der Grenze zwischen einem Trennbereich (S₁, ZS₂) und einem aktiven Bereich (ZA₁, ZA₂) aufweist, die geeignet ist, um einen Formgebungsfehler am Scheitel eines Winkels (A₂) der anderen Oberfläche auszugleichen, der direkt an der Grenze angeordnet ist.

6. Linse nach einem der vorhergehenden Ansprüche, wobei die aktiven Bereichen (ZA₁, ZA₂) und die Trennbereiche (ZS₁, ZS₂) im Inneren von jeder der ersten und der zweiten Oberfläche (S₁, S₂) konzentrisch sind.

7. Linse nach einem der vorhergehenden Ansprüche, wobei sich der Teil der optischen Leistung der Linse, die von der ersten und der zweiten Oberfläche (S₁, S₂) erzeugt wird, aus einer Refraktion oder aus einer Diffraktion des Lichtes in den aktiven Bereichen (ZA₁, ZA₂) und den Trennbereichen (ZA₁, ZA₂) von jeder Oberfläche ergibt.

8. Linse nach einem der vorhergehenden Ansprüche vom Typ der ophtalmischen Linse, einschließlich Brillenglas, Kontaktlinse und intraokularem Implantat.

9. Linse nach einem der vorhergehenden Ansprüche, umfassend mindestens zwei getrennte Linsenteile (1a, 1b), wobei ein erster der Linsenteile die erste optische Oberfläche (S₁) bildet und wobei ein zweiter Teil der Linsenteile die zweite optische Oberfläche (S₂) bildet, und wobei der erste und der zweite Linsenteil derart ausgebildet sind, dass die erste und die zweite optische Oberfläche einander zugewandt sind.

10. Linse nach Anspruch 9, wobei der erste und der zweite Linsenteil (1a, 1b) in einem peripheren Bereich der Linse komplementäre Muster (4) aufweisen, wobei die Muster geeignet sind, um eine Ausrichtung des ersten und des zweiten Linsenteils zueinander bei einem Zusammenfügen der Linsenteile zum Erstellen der Linse zu erzeugen.

11. Anordnung zur Formgebung von einer Linse, umfassend zwei Master-Oberflächen, die geeignet sind, um jeweils die erste und die zweite optische Oberfläche (S₁, S₂) von einer Linse (1) nach einem der Ansprüche 1 bis 10 zu bilden.

12. Anordnung zur Formgebung nach Anspruch 11, die ein Paar Prägematrizen oder ein Paar Formeinsätze (2, 3) bildet, wobei die Prägematrizen oder Formeinsätze jeweils die erste und die zweite Master-Oberfläche aufweisen.

## Claims

1. Lens (1) comprising at least two optical surfaces (S₁, S₂) each extending transversely relative to an optical axis (A-A) of the lens, so that light rays that pass through the lens pass through one then the other of the two surfaces, each surface comprising active zones (ZA₁, ZA₂) inside of which a shape of said surface is adapted to produce part at least of the optical power of the lens for rays that pass through said active zones, with a sagittal height discontinuity of each surface between two different active zones of said surface,
limits of the active zones (ZA₁, ZA₂) of a first of the two surfaces being located in line with limits of the active zones of the other of said two surfaces,
said first and second surfaces (S₁, S₂) furthermore comprising separating zones (ZS₁, ZS₂) arranged so that each of the first and second surfaces consists of an alternation between the active zones and the separating zones,
each active zone (ZA₁, ZA₂) of one of the first and second surfaces (S₁, S₂) being in line with a separating zone (ZS₂, ZS₁) of the other of said first and second surfaces, and
the shape of at least one of the first and second surfaces (S₁, S₂) in at least one of the separating zones (ZS₁, ZS₂) is adapted to increase at least one of the following angles:
- a first angle (A₁) formed by said surface (S₁, S₂) in said separating zone (ZS₁, ZS₂) with a direction of a sagittal height jump located at a limit between said separating zone and an active zone (ZA₁, ZA₂) adjacent to said separating zone,
- a second angle (A₂) formed by said surface (S₁, S₂) at a limit between said separating zone (ZS₁, ZS₂) and an active zone (ZA₁, ZA₂) adjacent to said separating zone, and
- a third angle (A₃) formed by the other surface (S₂, S₁) at a limit of the active zone (ZA₂, ZA₁) of said other surface that is located in line with said separating zone (ZS₁, ZS₂), said third angle being formed by said other surface in said active zone with a direction of a sagittal height jump located at the limit of said active zone,
relative to an angle (A₁₀, A₂₀, A₃₀) located at the same location of the lens (1) and formed by an imaginary surface (S₀) obtained by subtracting respective sagittal heights of the first and second surfaces (S₁, S₂), said imaginary surface thus comprising sagittal height jumps located in line with limits between the separating zones and active zones, and the angle (A₁₀, A₂₀, A₃₀) formed by the imaginary surface being measured between said imaginary surface and the sagittal height jump direction, and being acute,
the lens being **characterized in that** a sagittal height variation amplitude of each of the first and second surfaces (S₁, S₂) in at least one of the separating zones (ZS₁, ZS₂) of said surface is smaller than 25% of a sagittal height variation amplitude of the other surface in the active zone (ZA₁, ZA₂) located in line with said separating zone.

2. Lens according to Claim 1 in which, for at least one of the first and second surfaces (S₁, S₂), said surface in at least one of the separating zones (ZS₁, ZS₂) is recessed relative to said surface in the active zones (ZA₁, ZA₂) adjacent to said separating zone.

3. Lens according to Claim 2 in which, for each of the first and second surfaces (S₁, S₂), said surface in each separating zone (ZS₁, ZS₂) is recessed relative to said surface in the active zones (ZA₁, ZA₂) adjacent to said separating zone.

4. Lens according to Claim 1, in which each of the first and second surfaces (S₁, S₂) is planar or corresponds to a cambered base of said lens inside each separating zone (ZS₁, ZS₂).

5. Lens according to any one of the preceding claims, in which, for at least one of the first and second surfaces (S₁, S₂), said surface has a shape, on at least one side of a sagittal height jump of said surface, at the limit between a separating zone (ZS₁, ZS₂) and an active zone (ZA₁, ZA₂), adapted to compensate a forming defect at the tip of an angle (A₂) of the other surface located in line with said limit.

6. Lens according to any one of the preceding claims, in which the active zones (ZA₁, ZA₂) and the separating zones (ZS₁, ZS₂) are concentric inside each of the first and second surfaces (S₁, S₂).

7. Lens according to any one of the preceding claims, for which the part of the optical power of said lens that is produced by the first and second surfaces (S₁, S₂) results from a refraction or a diffraction of light in the active zones (ZA₁, ZA₂) and the separating zones (ZA₁, ZA₂) of each surface.

8. Lens according to any one of the preceding claims, of the ophthalmic lens type, including therein spectacle glasses, contact lenses and ocular implants.

9. Lens according to any one of the preceding claims, comprising at least two separate lens parts (1a, 1b), a first of said lens parts forming the first optical surface (S₁), and a second of said lens parts forming the second optical surface (S₂), and the first and second lens parts being arranged so that said first and second optical surfaces are turned toward each other.

10. Lens according to Claim 9, in which the first and second lens parts (1a, 1b) have complementary patterns (4) in a peripheral zone of the lens, said patterns being adapted to produce an alignment of said first and second lens parts relative to each other during assembly of said lens parts to form the lens.

11. Assembly for forming a lens, comprising two master-surfaces adapted to form the first and second optical surfaces (S₁, S₂), respectively, of a lens (1) according to any one of Claims 1 to 10.

12. Forming assembly according to Claim 11, consisting of a pair of embossing matrices or a pair of molding inserts (2, 3), said embossing matrices or molding inserts comprising the first and second master-surface, respectively.
